# EUROPÄISCHE PATENTSCHRIFT

(11) **EP 2 253 610 B1**
(45) Veröffentlichungstag und Bekanntmachung des Hinweises auf die Patenterteilung: **20.11.2013**
(21) Anmeldenummer: 10166545.3
(22) Anmeldetag: 05.09.2008
(51) Int. Cl.: C07C 227/08, C07C 229/30, C07D 213/38, C07D 213/61, C07D 307/58, C07D 405/12

(54) **Verfahren zur Herstellung von Enaminocarbonyl-Verbindungen und deren Verwendung zur Herstellung von 4-Aminobut-2-enoliden**
Method for manufacturing of enaminocarbonyl-compounds and their use in preparing 4-aminobut-2-enolids
Procédé de fabrication de composés enaminocarbonyle ainsi que leur utilisation pour la préparation de composés 4-aminobut-2-énoïque

(30) Priorität: 18.09.2007 EP 07116638
(43) Veröffentlichungstag der Anmeldung: 24.11.2010
(62) Teilanmeldung aus: 08801864.3
(73) Patentinhaber: Bayer CropScience AG, 40789 Monheim (DE)
(72) Erfinder: Lui, Norbert, Dr., 51519 Odenthal (DE); Heinrich, Jens-Dietmar, 51399 Burscheid (DE)

(56) Entgegenhaltungen:
- H BÖHME: "Kondensationsprodukte aus gamma-Chloracetessigester und primären aromatischen oder heteroaromatischen Aminen" CHEM BER, Bd. 109, 1976, Seiten 26-29, XP002473286
- MOMOSE T ET AL: "2(3H)- AND 2(5H)-FURANONES. III. AN EFFICIENT SYNTHESIS AND THE ESCHENMOSER-MANNICH REACTION OF N-SUBSTITUTED 4-AMINO-2 (5H)-FURANONES" HETEROCYCLES, Bd. 27, Nr. 8, 1988, Seiten 1907-1923, XP000946112 ISSN: 0385-5414
- ANZINI M ET AL: "MOLECULAR BASIS OF PERIPHERAL VS CENTRAL BENZODIAZEPINE RECEPTOR SELECTIVITY IN A NEW CLASS OF PERIPHERAL BENZODIAZEPINE RECEPTOR LIGANDS RELATED TO ALPIDEM" JOURNAL OF MEDICINAL CHEMISTRY, AMERICAN CHEMICAL SOCIETY. WASHINGTON, US, Bd. 39, Nr. 21, 1996, Seiten 4275-4284, XP002948777 ISSN: 0022-2623
- PILAR PUEBLA ET AL: "A Convenient Method for the Synthesis of Six-membered Heterocyclic Enaminones" JOURNAL OF HETEROCYCLIC CHEMISTRY, PROVO, UT, US, Bd. 36, 1999, Seiten 1097-1100, XP009097372 ISSN: 0022-152X

## Beschreibung

Die vorliegende Erfindung betrifft ein Verfahren zur Herstellung von 4-Amino-but-2-enoliden sowie von entsprechenden Intermediaten bzw. Ausgangsverbindungen, welche in dem erfindungsgemäßen Verfahren durchlaufen bzw. verwendet werden. Weiterer Gegenstand der vorliegenden Erfindung sind Verfahren zur Herstellung der entsprechenden Intermediate bzw. Ausgangsverbindungen.

Bestimmte substituierte 4-Aminobut-2-enolid-Verbindungen sind als insektizid wirksame Verbindungen aus der EP 0 539 588 A1 bekannt. Darüber hinaus beschreiben auch die Patentanmeldungen WO 2007/115644, WO 2007/115643 und WO 2007/115646 entsprechende insektizid wirksame 4-Aminobut-2-enolid-Verbindungen.

H. Böhme "Kondensationsprodukte aus gamma-Chloracetatessigester und primären aromatischen oder heteroaromatischen Aminen", Arch. Pharm. 310, 1976, Seiten 26 - 29 offenbart die Reaktion von Anilin mit gamma-Chloracetoacetat (Ia) in Substanz, d.h. ohne Lösungsmittel. Die Komponenten werden dabei in Gegenwart von Schwefelsäure vereint und mehrere Tage bei erhöhter Temperatur gerührt. Die erfindungsgemäße Reaktion wird jedoch nicht offenbart.

Im Allgemeinen werden Enaminocarbonylverbindungen aus Tetronsäure und einem Amin gemäß dem nachfolgenden Schema 1 synthetisiert. Diese Vorgehensweise ist beispielsweise in EP 0 539 588 A1 sowie in Heterocycles Vol. 27, Nr. 8, Seite 1907 bis 1923 (1988) beschrieben.

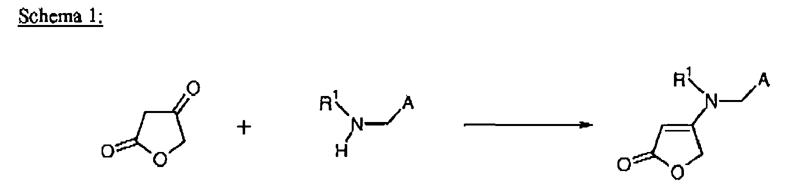

Nachteilig an diesem Verfahren ist insbesondere, dass man wasserfreie Tetronsäure als Ausgangsverbindung benötigt, deren Herstellung aufwendig und kostenintensiv ist.

So wird Tetronsäure im Allgemeinen ausgehend von Acetessigester über eine Bromierung und anschließende Hydrierung hergestellt (vgl. Synthetic Communication, 11(5), Seiten 385 bis 390 (1981)). Die Gesamtausbeute an Tetronsäure ausgehend von Acetessigester ist dabei kleiner als 40 %, was das Verfahren unter industriellem Gesichtspunkt wenig attraktiv macht.

In der CH-PS 503 722 ist ein weiteres Verfahren zur Herstellung von Tetronsäure beschrieben. Dabei wird 4-Chloracetessigester mit einem aromatischen Amin zum 3-Arylaminocrotonlacton umgesetzt und anschließend wird die Tetronsäure durch Behandlung mit Mineralsäuren freigesetzt. Der Nachteil an diesem Verfahren besteht darin, dass die Isolierung der Tetronsäure nur durch Hochvakuumsublimation möglich ist, was auch dieses Verfahren unter industriellem Gesichtspunkt wenig attraktiv macht.

Ein weiteres Verfahren zur Herstellung von Tetronsäure ist in der EP 0 153 615 A beschrieben, in welchem von 2,4-Dichloracetessigestern ausgegangen wird. Dieses ebenfalls mehrstufige und aufwändige Verfahren liefert die gewünschte Verbindung ebenfalls nur mit einer mäßigenGesamtausbeute von 65 %.

In Tetrahedron Letters, Nr. 31, Seiten 2683 und 2684 (1974) ist die Herstellung von Tetronsäure und einer entsprechenden Enaminocarbonylverbindung beschrieben. Die dort beschriebene Synthese ist in folgendem Schema 2 wiedergegeben. Als Edukt wird dabei Acetylendicarbonsäuredimethylester eingesetzt.

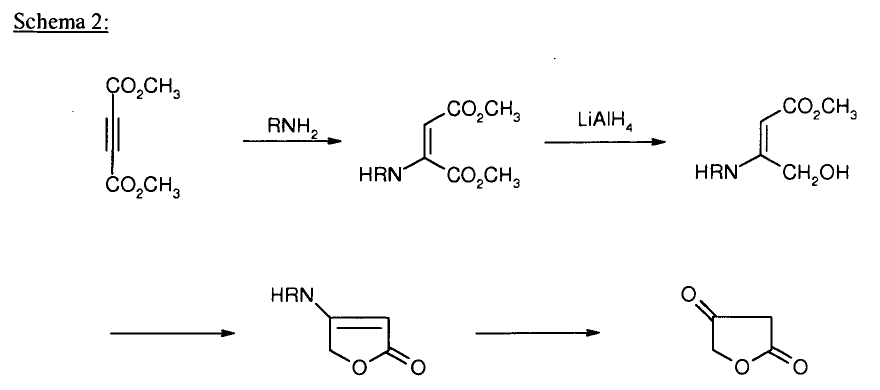

Nachteilig an diesem Verfahren sind die geringe Gesamtausbeute von nur 30 % sowie das Erfordernis, kostenintensive Edukte, beispielsweise Lithiumaluminiumhydrid (LiAlH4), als Reagenzien verwenden zu müssen.

Ferner ist aus dem Stand der Technik ein Verfahren zur Herstellung von 4-Aminobut-2-enoliden ausgehend von Methyltetronat bekannt (J. Heterocyclic Chem., 21, 1753 (1984)). Für dieses Verfahren wird als Ausgangsmaterial der kostenintensive 4-Brom-3-methoxy-but-3-encarbonsäureester verwendet.

Ein weiteres Verfahren geht von einem 4-Chloracetessigester aus, der mit Aminen umgesetzt wird (Heterocycles, Vol. 27, Nr. 8, 1988, Seiten 1907 bis 1923). Die Reaktion zum Aminofuran wird in einem Schritt durchgeführt. Dabei wird das Amin mit Eisessig zu einer Lösung von 4-Chloracetessigester in Benzol gegeben und die resultierende Mischung wird mehrere Stunden unter Rückfluss erhitzt. Die Ausbeuten an 4-Methylamino-2(5H)-furanon in dieser Synthese betragen nur 40%.

Aus EP 0 123 095 A ist ein Verfahren bekannt, in welchem Tetronsäureamid aus 3-Amino-4-acetoxycrotonsäureester hergestellt wird. 3-Amino-4-acetoxycrotonsäureester ist kostenintensiv und aufwändig herzustellen, so dass eine wirtschaftliche Synthese mit diesem Verfahren nicht möglich ist.

Ein weiteres Verfahren zur Herstellung von Tetronsäure ausgehend von Malonestern und Chloracetylchlorid ist aus J. Chem. Soc., Perkin Trans. 1 (1972), Nr. 9/10, Seiten 1225 bis 1231 bekannt. Dieses Verfahren liefert die gewünschte Zielverbindung mit einer Ausbeute von nur 43 %. In der vorstehend erwähnten, Internationalen Patentanmeldung WO 2007/115644 wird die Herstellung von 4-Aminobut-2-enoliden, beispielsweise von 4-[[(6-Chlorpyridin-3-yl)methyl](3,3-dichlorprop-2-en-1-yl)amino]furan-2(5H)-on durch Umsetzung von 4-[[(6-Chlorpyridin-3-yl)methyl]amino]furan-2(H)-on mit 3-Brom-1,1-dichlorprop-1-en, beschrieben (vgl. Herstellungsbeispiel, Verfahren 2, Beispiel (3)). Die PCT/EP2007/002386 beschreibt auch die Herstellung von 4-Aminobut-2-enoliden, beispielsweise von 4-[[(6-Chlorpyridin-3-yl)methyl](3,3-dichlorprop-2-en-1-yl)amino]furan-2(5H)-on durch Umsetzung von 4-[[2-Fluorethyl)amino]furan-2(5H)-on mit 2-Chlor-5-chlormethyl-pyridin (vgl. Herstellungsbeispiele, Verfahren 3, Beispiel (4)). Die Reaktionen werden vorzugsweise mit Hydriden des Lithiums oder Natriums durchgeführt. Diese Substrate sind im Allgemein kostenintensiv und gleichzeitig aus Sicherheitsgründen nur schwer zu handhaben. Im erfindungsgemäßen Verfahren werden dagegen preiswerte und sicherheitstechnologisch einfacher handhabbare Basen, z.B. NaOH verwendet.

Die Aufgabe der vorliegenden Erfindung bestand in der Bereitstellung eines neuen, wirtschaftlichen Verfahrens zum Herstellen von 4-Aminobut-2-enolidverbindungen und zum Herstellen von Ausgangsverbindungen für dieses Verfahren.

Gegenstand der vorliegenden Erfindung ist daher ein Verfahren zum Herstellen von Verbindungen der allgemeinen Formel (IVa) gemäß Anspruch 1 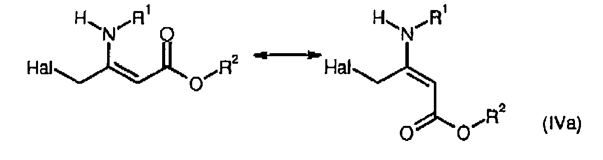 in welcher
- R¹: für Methyl, Ethyl, n-Propyl, n-Prop-2-enyl, n-Prop-2-inyl, Cyclopropyl, Methoxyethyl, 2-Fluorethyl, oder 2,2-Difluorethyl steht,
- R²: für Methyl oder Ethyl steht und
- Hal: für Chlor steht,
**dadurch gekennzeichnet, dass**
4-Halogenacetessigester der allgemeinen Formel (II) 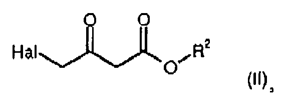 in welcher R² und Hal die oben genannte Bedeutung haben,
mit einem Amin der Formel (IIIa) 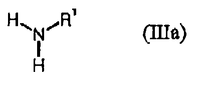 in welcher R¹ die oben genannte Bedeutung hat,
zur Verbindung der Formel (IVa) umgesetzt wird.

Weiterhin wird offenbart ein Verfahren zum Herstellen von 4-Aminobut-2-enoliden der allgemeinen Formel (I) 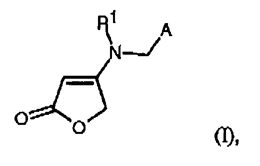 in welcher
A die unten genannten und R¹ die oben genannten Bedeutungen haben,
**dadurch gekennzeichnet, dass** 4-Halogenacetessigester der allgemeinen Formel (II) 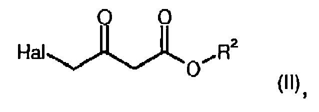 in welcher R² und Hal die oben genannte Bedeutung haben, mit
a) einem Amin der Formel (IIIa) 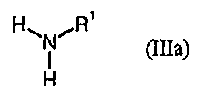 in welcher R¹ die oben genannte Bedeutung hat,
   zu einer Verbindung der Formel (IVa) 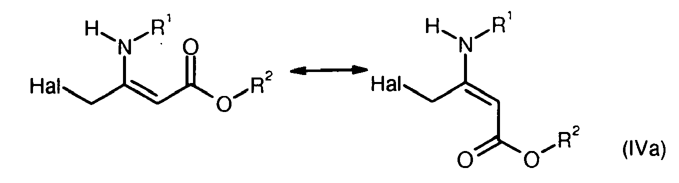 umgesetzt wird, anschließend die Verbindung der Formel (IVa) in Gegenwart eines Lösungsmittels thermisch zu einer Verbindung der Formel (Va) cyclisiert wird 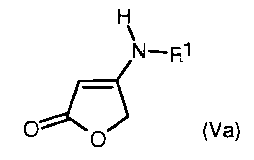 und die Verbindung der Formel (Va) im letzten Schritt mit einer Verbindung der Formel (VIa)

   A-CH₂-E (VIa)

   wobei A und E die unten genannte Bedeutung hat, zur Verbindung der Formel (I) umgesetzt wird.

A steht für den Rest 6-Fluor-pyrid-3-yl, 6-Chlor-pyrid-3-yl, 6-Brom-pyrid-3-yl, 2-Chlor-1,3-thiazol-5-yl, 5.Fluor-6-chlor-pyrid-3-yl,5-Fluor-6-brom-pyrid-3-yl oder 5,6-Dichlor-pyrid-3-yl steht; undAbgangsgruppen E sind Chlor, Brom oder Jod oder Mesylat, Tosylat oder SO₂Me.

Überraschenderweise lassen sich die 4-Aminobut-2-enolide der Formel (I) unter den erfindungsgemäßen Bedingungen mit sehr guten Ausbeuten in hoher Reinheit herstellen, womit das erfindungsgemäße Verfahren die oben genannten Nachteile überwindet. Gegenüber dem aus dem Stand der Technik bekannten Verfahren, das von 4-Chloracetessigester ausgeht und diesen mit Aminen umsetzt (Heterocycles, Vol. 27, No. 8, 1988, 1907 - 1923) konnten die Ausbeuten durch das erfindungsgemäße Verfahren verdoppelt werden. Auch gegenüber dem in der WO 2007/115644 beschriebenen Verfahren zur Herstellung von 4-Aminobut-2-enoliden (vgl. oben) konnte die Ausbeute durch das erfindungsgemäße Verfahren wesentlich erhöht werden.

Ein weiterer Gegenstand der vorliegenden Erfindung sind Verbindungen der Formel (IVa) 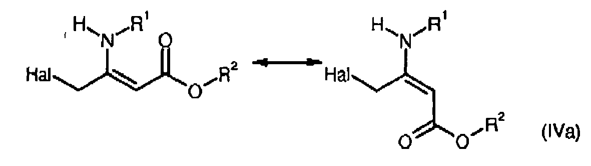 in welcher
Hal für Chlor steht und
R¹, und R² wie oben definiert sind.

Das offenbarte Verfahren kann anhand des folgenden Schemes 3a erläutert werden:

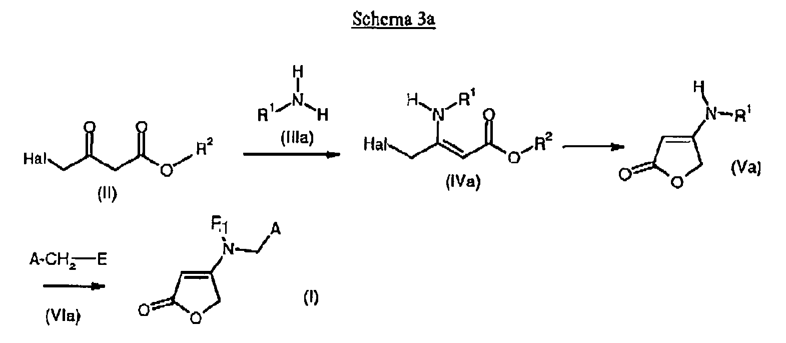

Die gemäß der vorliegenden Erfindung verwendeten 4-Halogenacetessigester sind Verbindungen gemäß der allgemeinen Formel (II) 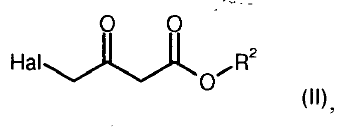 in welcher R² für Methyl oder Ethyl, und Hal für Cl, steht.

Die 4-Halogenacetessigsäureesterverbindungen sind kommerziell erhältlich oder können nach literaturbekannten Verfahren leicht hergestellt werden (Organic Syntheses (1973), 53, 1882; US Pat. No 4,468,356).

Die Umsetzung des 4-Halogenacetessigesters mit dem Amin der Formel (IIIa) zur Verbindung mit der Formel (IVa) kann in Gegenwart von Toluol oder Mischungen aus Toluol und Ethanol.

Amine der Formel (IIIa) sind kommerziell erhältlich oder können nach literaturbekannten Verfahren hergestellt werden (vgl. 2-Fluor-ethylamin: US-Pat. 4030994 (1977); 3-Fluor-n-propylamin: US 6252087 B1; 3,3-Difluor-prop-2-enylamin Hydrochlorid: WO 2001/007414 A1; 3,3-Dichlor-prop-2-enylamin: DE 2747814; 2-Chlor-2-fluor-cyclopropylamin, 2,2-Dichlorcyclopropylamin: K. R. Gassen, B. Baasner, J. Fluorine Chem. 49, 127-139, 1990; Verbindungen der Formel (IIIa) oder (IIIb) in welcher R¹ für Alkyl steht, primäre Amine: vgl. z. B. Houben-Weyl, Methoden der Organischen Chemie, Bd. XI/1, 4. Aufl. 1957, Georg Thieme Verlag Stuttgart, S. 648; M. L. Moore in "The Leuckart Reaction" in: Organic Reactions, Bd. 5, 2. Aufl. 1952, New York, John Wiley & Sons, Inc. London).

Die Verbindungen der Formel (IVa) liegen als E/Z Gemisch vor.

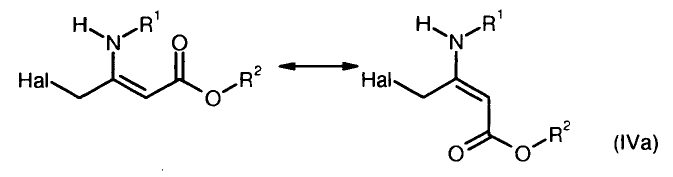

Zur Umsetzung der Verbindung der Formel (II) mit dem Amin der Formel (IIIa) kann ggf. eine Lewis-Säure als Katalysator zugesetzt werden. Beispiele hierfür sind Essigsäure, p-Toluolsulfonsäure, Trifluoressigsäure. Bevorzugt verwendet wird Essigsäure.

Das Amin mit der Formel (IIIa) kann auch in Form des Salzes einsetzt werden. Dadurch kann der Zusatz an Lewis-Säure entfallen oder reduziert werden.

Die Umsetzung der Verbindung der Formel (II) mit dem Amin der Formel (IIIa) wird bei Normaldruck und Temperaturen von 10°C bis 40°C, am meisten bevorzugt bei 10°C bis 30°C durchgeführt. Ein Gegenstand der vorliegenden Erfindung ist daher auch ein Verfahren zur Herstellung von Verbindungen der Formel (IVa) oder zur Herstellung von Verbindungen der Formel (I), wobei die Umsetzung der Verbindung der Formel (II) mit dem Amin der Formel (IIIa) bei 10°C bis 40°C bei Normaldruck erfolgt. Die Reaktionsdauer beträgt zwischen 0,5 und 10 Stunden, längere Reaktionszeiten wirken sich nicht nachteilig aus. Das Lösungsmittel kann durch Abdestillieren oder im Vakuum im Temperaturbereich von 20°C bis 35°C entfernt werden.

Die Cyclisierung der Verbindung der Formel (IVa) zur Verbindung der Formel (Va) kann in einem inerten Lösungsmittel durchgeführt werden. Geeignete Lösungsmittel sind beispielsweise ausgewählt aus der Gruppe bestehend aus aliphatischen und aromatischen Kohlenwasserstoffen, wie n-Hexan, Benzol, Toluol und Xylol, die durch Fluor- und Chloratome substituiert sein können, wie Methylenchlorid, Dichlormethan, Trichlormethan, Tetrachlorkohlenstoff, Fluorbenzol, Chlorbenzol oder Dichlorbenzol; Ethern, wie Diphenylether Methyl-tert-butylether, Isopropylethylether, Dioxan, Dimethylglycol oder THF; und Nitrilen wie Methylnitril, Butylnitril oder Phenylnitril; wobei Toluol bevorzugt ist.

Die Cyclisierung der Verbindung der Formel (IVa) zur Verbindung der Formel (Va) kann im Vakuum, bei Normaldruck oder unter Überdruck und bei Temperaturen von -20°C bis 200°C durchgeführt werden, bevorzugt bei 40°C - 150°C. Ein Gegenstand der vorliegenden Erfindung ist daher auch ein Verfahren zur Herstellung von Verbindungen der Formel (I), wobei die thermische Cyclisierung der Verbindung der Formel (IVa) zur Verbindung der Formel (Va) und der Verbindung der Formel (IVb) zur Verbindung der Formel (Vb) bei 40°C bis 150°C erfolgt. Die Reaktionsdauer beträgt zwischen 1 h und 10 Stunden, längere Reaktionszeiten wirken sich nicht nachteilig aus.

Die Isolierung der Verbindung der Formel (Va) erfolgt durch Kristallisation oder durch Entfernen des Lösungsmittels.

Die Herstellung der 4-Aminobut-2-enolide der Formel (I) erfolgt durch Umsetzung der Verbindung der Formel (Va) mit Verbindungen der Formel (VIa).

Die Verbindungen der Formel (VIa) sind z. T. kommerziell erhältlich, teilweise bekannt und können nach bekannten Methoden erhalten werden (z. B. 2-Chlor-5-chlormethyl-1,3-thiazol: DE 3 631 538 (1988), EP 446 913 (1991), EP 780 384 (1997), EP 775 700 (1997), EP 794 180 (1997), WO 9 710 226 (1997); 6-Chlor-3-chlormethyl-pyridin: DE 3 630 046 A1 (1988), EP 373 464 A2 (1990), EP 373 464 A2 (1990), EP 393 453 A2 (1990), EP 569 947 A1 (1993); 6-Chlor-3-brommethyl-pyridin: I. Cabanal-Duvillard et al., Heterocycl. Commun. 5, 257-262 (1999); 6-Brom-3-chlormethyl-pyridin, 6-Brom-3-hydroxymethyl-pyridin: US-Pat. 5 420 270 A (1995); 6-Fluor-3-chlormethyl-pyridin: J. A. Pesti et al., J. Org. Chem. 65, 7718-7722 (2000); 6-Methyl-3-chlormethyl-pyridin: EP 302389 A2, E. v der Eycken et al., J. Chem. Soc., Perkin Trans 2 5, 928-937 (2002); 6-Trifluormethyl-3-chlormethyl-pyridin: WO 2004/082616 A2; 2-Chlor-5-chlormethyl-pyrazin: JP 05239034 A2).

Allgemeine Wege zur Herstellung von Verbindungen der Formel (VIa) sind im folgenden Schema 4 wiedergegeben. 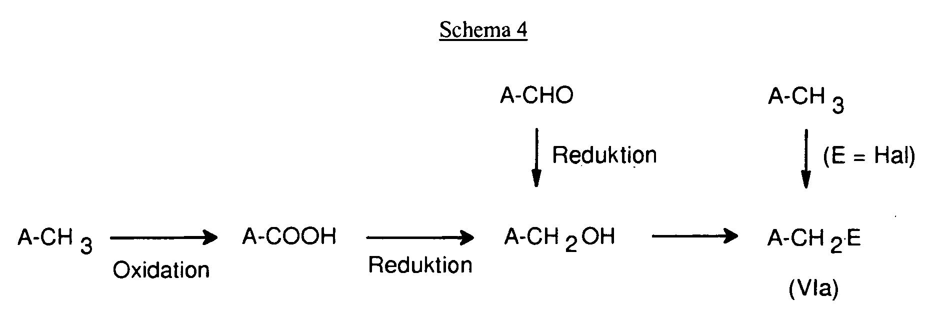 E = Hal, beispielsweise Chlor, Brom, Iod; Mesylat, Tosylat oder SO₂Me
A = wie oben definiert

Beispielsweise können die heterocyclischen Carbonsäuren (A-COOH) nach literaturbekannten Methoden in die entsprechenden heterocyclischen Hydroxymethylverbindungen (A-CH₂-OH) überführt werden, die anschließend nach literaturbekannten Methoden zu aktivierten heterocyclischen Hydroxymethylverbindungen (A-CH₂-E, E = Tosylat, Mesylat) oder heterocyclischen Halogenmethylverbindungen (A-CH₂-E, E = Hal) umgesetzt werden. Letztere können auch aus entsprechenden Methylgruppe-haltigen Heterocyclen (A-CH₃) unter Verwendung von geeigneten literaturbekannten Halogenierungsmitteln erhalten werden.

Als Abgangsgruppe E sind Gruppen ausgewählt unter Chlor, Brom, oder Jod, oder Mesylat, Tosylat oder SO₂Me als geeignete Abgangsgruppen genannt. Bevorzugt sind Chlor, Brom und Mesylat.

Die Reaktion erfolgt vorzugsweise in Gegenwart einer Base. Geeignete Basen sind organische und anorganische Basen, die üblicherweise in solchen Reaktionen verwendet werden. Vorzugsweise werden Basen verwendet, die beispielhaft ausgewählt sind aus der Gruppe bestehend aus Hydriden, Hydroxiden, Amiden, Alkoholaten, Acetaten, Fluoriden, Phosphaten, Carbonaten und Hydrogencarbonaten von Alkali- oder Erdalkalimetallen. Besonders bevorzugt sind dabei Natriumamid, Natriumhydrid, Lithiumdiisopropylamid, Natriummethanolat, Kalium-*tert*-butanolat, Natriumhydroxid, Kaliumhydroxid, Natriumacetat, Natriumphosphat, Kaliumphosphat, Kaliumfluorid, Caesiumfluorid, Natriumcarbonat, Kaliumcarbonat, Kaliumhydrogencarbonat, Natriumhydrogencarbonat und Caesiumcarbonat. Ganz besonders bevorzugt sind Natriumhydroxid und Kaliumhydroxid. Ein Gegenstand der vorliegenden Erfindung ist daher auch ein Verfahren zur Herstellung von Verbindungen der Formel (I), wobei die Umsetzung der Verbindung der Formel (Va) mit der Verbindung der Formel (VIa) zur Verbindung der Formel (I) in Gegenwart von Natriumhydroxid oder Kaliumhydroxid erfolgt. Außerdem sind tertiäre Amine, wie Trimethylamin, Triethylamin, Tributylamin, N,N-Dimethylanilin, N,N-Dimethylbenzylamin, Pyridin, Alkylpyridine, wie 2-Methyl-5-Ethylpyridin, N-Methylpiperidin, N-Methylpyrolidon, N,N-Dimethylaminopyridin, Diazabicyclooctan (DABCO), Diazabicyclononen (DBN) und Diazabicycloundecen (DBU) bevorzugt.

Das molare Verhältnis von Base zu eingesetzten Tetronamid der Formel (Va) beträgt beispielsweise 0,5 - 10, bevorzugt 0,9 - 6, besonders bevorzugt 1,0 - 2. Der Einsatz größerer Mengen an Base ist grundsätzlich möglich, führt jedoch zu keiner bevorzugten Ausführungsform und ist aus wirtschaftlichen Gründen nachteilig.

Das molare Verhältnis von Alkylierungsmittel der Formel (VIa) zu eingesetztem Tetronamid der Formel (Va) beträgt beispielsweise 0,5 - 3, bevorzugt 0,9 - 2, besonders bevorzugt 1,0 - 1,5. Der Einsatz größerer Mengen an Alkylierungsmittel ist grundsätzlich möglich, führt jedoch zu keiner bevorzugten Ausführungsform und ist aus wirtschaftlichen Gründen nachteilig.

Gegebenenfalls kann bei der Reaktion des Tetronamids der Formel (Va) mit dem Alkylierungsmittel der Formel (VIa) ein Phasentransferkatalysator eingesetzt werden, wie quartäre Ammonium- oder Phosphoniumverbindungen.

Die Reaktion des Tetronamides der Formel (Va) mit dem Alkylierungsmittel der Formel (VIa) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden aliphatische, alicyclische oder aromatische Kohlenwasserstoffe, wie Petrolether, Hexan, Heptan, Cyclohexan, Methylcyclohexan, Benzol, Toluol, Xylol oder Decalin; halogenierte Kohlenwasserstoffe, wie Chlorbenzol, Dichlorbenzol, Dichlormethan, Chloroform, Tetrachlorkohlenstoff, Dichlorethan oder Trichlorethan; Ether, wie Diethylether, Diisopropylether, Methyl-tert-butylether (MTBE), Methyl-tert-amylether, Dioxan, Tetrahydrofuran, Methyl-THF, 1,2-Dimethoxyethan, 1,2-Diethoxyethan oder Anisol; Nitrile, wie Acetonitril, Propionitril, n- oder iso-Butyronitril oder Benzonitril; Amide, wie N,N-Dimethylformamid (DMF), N,N-Dimethylacetamid, N-Methylformanilid, N-Methylpyrrolidon (NMP); oder Mischungen dieser.

Die Reaktion des Tetronamides der Formel (Va) mit dem Alkylierungsmittel der Formel (VIa) kann im Vakuum, bei Normaldruck oder unter Überdruck und bei Temperaturen von 0°C bis 150°C durchgeführt werden, vorzugsweise erfolgt die Reaktion bei Normaldruck und Temperaturen von 10 bis 90°C. Die Reaktionsdauer beträgt zwischen 1 h und 30 Stunden, längere Reaktionszeiten wirken sich nicht nachteilig aus.

Die abschließende Reinigung der 4-Aminobut-2-enoliden der Formel (I) kann durch übliche Reinigungsverfahren erfolgen. Vorzugsweise erfolgt die Reinigung durch Kristallisation.

Ein weiterer Gegenstand der vorliegenden Erfindung sind die Verbindungen der Formeln (IVa), wobei Hal für Cl steht sowie deren Verwendung gemäß Anspruch 4 und 5:

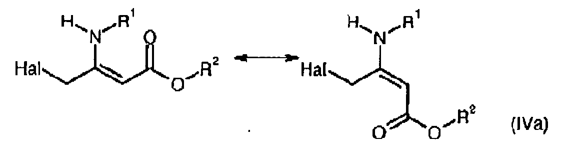

Die Verfahren zur Herstellung von 4-Aminobut-2-enoliden der Formel (I) und von Ausgangsprodukten zu deren Herstellung wird in den nachstehenden Beispielen beschrieben, welche die obige Beschreibung weiter illustrieren.

### Herstellungsbeispiele:

### Beispiel 1 (erfindungsgemäß)

Zu einer Lösung von 300 g 4-Chloracetessigsäureethylester in 519 ml Toluol und 200 ml Ethanol werden 20,8 ml Essigsäure zugesetzt. Bei 10°C - 30°C werden unter Kühlung 188,7 g einer 33%igen Methylamin-Lösung in Ethanol zugetropft. Anschließend wird 8 h bei Raumtemperatur gerührt und das Lösungsmittel im Vakuum bei Temperaturen von bis zu 35°C entfernt. Man erhält 328 g 4-Chlor-3-(methylamino)but-2-encarbonsäureethylester in einer Reinheit von 91 % (dies entspricht 92 % Ausbeute).
¹H-NMR (CDCl₃, 298K) δ: 1,25 t (3H), 3,01 d (3H), 4,00 s (1H), 4,10 q (2H), 4,67 s + 4,96 s (1H) E/Z, 8,22 s (1H; NH)

### Beispiel 2 (erfindungsgemäß)

Zu einer Lösung von 30 g 4-Chloracetessigsäureethylester in 90 ml Toluol und 30 ml Ethanol werden 2 ml Essigsäure zugesetzt. Bei 10°C - 30°C werden unter Kühlung 8,9 g Ethylamin zugetropft. Anschließend wird 6 h bei Raumtemperatur gerührt und das Lösungsmittel im Vakuum bei Temperaturen von bis zu 35°C entfernt. Man erhält 37,7 g 4-Chlor-3-(ethylamino)but-2-encarbonsäureethylester in einer Reinheit von 88 % (dies entspricht 98 % Ausbeute).
¹H-NMR (DMSO d₆, 298K) δ: 1,16 m (6H), 2,81 q + 2,96 q (2H) E/Z, 3,32 q (2H), 4,32 s (2H), 4,43 s + 4,70 s (1H) E/Z 8,3 - 9,3 breit (1H,NH)

### Beispiel 3 (erfindungsgemäß)

Zu einer Lösung von 100 g 4-Chloracetessigsäureethylester in 300 ml Toluol und 100 ml Ethanol werden 7 ml Essigsäure zugesetzt. Bei 10°C - 30°C werden unter Kühlung 37,3 g n-Propylamin zugetropft. Anschließend wird 9 h bei Raumtemperatur gerührt und das Lösungsmittel im Vakuum bei Temperaturen von bis zu 35°C entfernt. Man erhält 140,6 g 4-Chlor-3-(n-propylamino)but-2-encarbonsäureethylester in einer Reinheit von 76 % (dies entspricht 88 % Ausbeute).
¹H-NMR (DMSO d₆, 298K) δ: 0,92 t (3H), 1,16 t (3H), 1,56 m (2H), 3,26 q (2H), 4,00 m (2H), 4,31 s (2H), 4,44 s + 4,72 s (1H) E/Z, 7,7 - 8,8 breit (1H,NH)

### Beispiel 4 (nicht erfindungsgemäß)

Zu einer Lösung von 100 g 4-Chloracetessigsäureethylester in 300 ml Toluol und 100 ml Ethanol werden 7 ml Essigsäure zugesetzt. Bei 10°C - 30°C werden unter Kühlung 38,3 g iso-Propylamin zugetropft. Anschließend wird 5 h bei Raumtemperatur gerührt und das Lösungsmittel im Vakuum bei Temperaturen von bis zu 35°C entfernt. Man erhält 141,8 g 4-Chlor-3-(iso-propylamino)but-2-encarbonsäureethylester in einer Reinheit von 70 % (dies entspricht 82 % Ausbeute).
¹H-NMR (DMSO d₆, 298K) δ: 1,16 t (3H), 1,21 d (6H), 3,26 q (2H), 4,00 m (1H), 4,34 s (2H), 4,44 s + 4,69 s (1H) E/Z, 7,7 - 9,0 breit (1H, NH)

### Beispiel 5 (nicht erfindungsgemäß)

Zu einer Lösung von 100 g 4-Chloracetessigsäureethylester in 300 ml Toluol und 100 ml Ethanol werden 6,7 ml Essigsäure zugesetzt. Bei 10°C - 30°C werden unter Kühlung 48,3 g n-Butylamin zugetropft. Anschließend wird 8 h bei Raumtemperatur gerührt und das Lösungsmittel im Vakuum bei Temperaturen von bis zu 35°C entfernt. Man erhält 136,6 g 4-Chlor-3-(n-butylamino)but-2-encarbonsäureethylester in einer Reinheit von 89 % (dies entspricht 94 % Ausbeute).
¹H-NMR (DMSO d₆, 298K) δ: 0,92 t (3H), 1,16 t (3H), 1,35 m (2H), 1,52 m (2H), 3,29 q (2H), 4,01 m (2H), 4,44 s + 4,72 s (1H) E/Z, 7,7-8,5 breit (1H,NH)

### Beispiel 6 (nicht erfindungsgemäß)

Zu einer Lösung von 100 g 4-Chloracetessigsäureethylester in 300 ml Toluol und 100 ml Ethanol werden 6,7 ml Essigsäure zugesetzt. Bei 10°C - 30°C werden unter Kühlung 47,4 g iso-Butylamin zugetropft. Anschließend wird 8 h bei Raumtemperatur gerührt und das Lösungsmittel im Vakuum bei Temperaturen von bis zu 35°C entfernt. Man erhält 140,8 g 4-Chlor-3-(iso-butylamino)but-2-encarbonsäureethylester in einer Reinheit von 79 % (dies entspricht 86 % Ausbeute).
¹H-NMR (DMSO d₆, 298K) δ: 0,91 t (3H), 0,93 t (3H), 1,17 t (3H), 1,78 m (1H), 3,12 t (2H), 4,02 q (2H), 4,31 s (2H), 4,44 s + 4,69 s (1H) E/Z , 8,0 - 8,5 breit (1H,NH)

### Beispiel 7 (erfindungsgemäß)

Zu einer Lösung von 100 g 4-Chloracetessigsäureethylester in 300 ml Toluol und 100 ml Ethanol werden 6,7 ml Essigsäure zugesetzt. Bei 10°C - 30°C werden unter Kühlung 46,5 g 2-Methoxyethylamin zugetropft. Anschließend wird 8 h bei Raumtemperatur gerührt und das Lösungsmittel im Vakuum bei Temperaturen von bis zu 35°C entfernt. Man erhält 140,8 g 4-Chlor-3-(2-methoxyethylamino)but-2-encarbonsäureethylester in einer Reinheit von 88 % (dies entspricht 95 % Ausbeute).
¹H-NMR (DMSO d₆, 298K) δ: 1,16 t (3H), 3,27 d (2H), 3,37 s (3H), 3,46 d (2H), 3,99 m (2H), 4,32 s (2H), 4,49 s + 4,72 s (1H) E/Z, 7,7-8,5 breit (1H,NH)

### Beispiel 8 (nicht erfindungsgemäß)

Zu einer Lösung von 100 g 4-Chloracetessigsäureethylester in 300 ml Toluol und 100 ml Ethanol werden 6,7 ml Essigsäure zugesetzt. Bei 10°C - 30°C werden unter Kühlung 65,7 g Benzylamin zugetropft. Anschließend wird 8 h bei Raumtemperatur gerührt und das Lösungsmittel im Vakuum bei Temperaturen von bis zu 35°C entfernt. Man erhält 157,8 g 4-Chlor-3-(benzylamino)but-2-encarbonsäureethylester in einer Reinheit von 50 % (dies entspricht 52 % Ausbeute).
¹H-NMR (DMSO d₆, 298K) δ: 1,11 t (3H), 3,95 q (2H), 4,24 d (2H), 4,34 s (2H), 4,44 s + 4,72 s ( 1H) E/Z , 7,20 - 7,40 m (5H), 8,3 - 8,8 breit (1H,NH)

### Beispiel 9 (nicht erfindungsgemäß)

Zu einer Lösung von 2,1 g 4-Chloracetessigsäureethylester in 12 ml Toluol werden 0,15 ml Essigsäure zugesetzt. Bei 10°C - 30°C werden unter Kühlung 2 g 2-Chlor-5-(aminomethyl)pyridin gelöst in 4 ml Ethanol zugetropft. Anschließend wird 8 h bei Raumtemperatur gerührt und das Lösungsmittel im Vakuum bei Temperaturen von bis zu 35°C entfernt. Man erhält 4,2 g 4-Chlor-3-([[6-chlorpyridin-3-yl]methyl]amino)but-2-encarbonsäureethylester in einer Reinheit von 86 % (dies entspricht 98 % Ausbeute).
¹H-NMR (CDCl₃, 298K) δ: 1,26 t (3H), 3,98 s (2H), 4,13 q (2H), 4,56 d (2H), 4,79 s + 4,99 s (1H) E/Z, 7,35 d (1H), 7,64 d (1H), 8,35 d (1H), 8,5 - 8,7 breit (1H,NH)

### Beispiel 10 (erfindungsgemäß)

Zu einer Lösung von 197,2 g 4-Chloracetessigsäureethylester in 1080 ml Toluol werden 13,3 ml Essigsäure zugesetzt. Bei 10°C - 30°C werden unter Kühlung 100 g 2,2-Difluorethylamin gelöst in 360 ml Ethanol zugetropft. Anschließend wird 8 h bei Raumtemperatur gerührt und das Lösungsmittel im Vakuum bei Temperaturen von bis zu 35°C entfernt. Man erhält 278 g 4-Chlor-3-(2,2-difluorethylamino)but-2-encarbonsäureethylester in einer Reinheit von 93% (dies entspricht 98% Ausbeute).
¹H-NMR (DMSO d₆, 298K) δ: 1,17 t (3H), 3,73 m (2H), 4,04 q (2H), 4,36 s (2H), 4,84 s (1H), 6,03 t + 6,16 t + 6,31 t (1 H, CHF₂), 8,2 - 8,4 t (breit) (1H,NH)

### Beispiel 11 (erfindungsgemäß)

328,8 g 4-Chlor-3-(methylamino)but-2-encarbonsäureethylester (Beispiel 1) werden in 519 g Toluol suspendiert und 4 h unter Rückfluss erhitzt. Anschließend wird auf 20°C abgekühlt, der Feststoff abfiltriert und mit 150 ml Toluol und mit 150 ml Ethanol gewaschen. Man erhält 142 g 4-(Methylamino)furan-2(5H)-on mit einer Reinheit von 95 % (dies entspricht 71% Ausbeute).
¹H-NMR (DMSO d₆, 298K) δ: 2,71 d (3H), 4,50 s (1H), 4,60 s (2H), 7,2 - 7,7 breit (1H,NH)

### Beispiel 12 (erfindungsgemäß)

37,5 g 4-Chlor-3-(ethylamino)but-2-encarbonsäureethylester (Beispiel 2) werden in 86,5 g Toluol suspendiert und 2 h unter Rückfluss erhitzt. Anschließend wird auf 20°C abgekühlt und das Lösungsmittel im Vakuum entfernt. Man erhält 25,5 g 4-(Ethylamino)furan-2(5H)-on mit einer Reinheit von 74 % (dies entspricht 75% Ausbeute).
¹H-NMR (DMSO d₆, 298K) δ: 1,12 t (3H), 3,07 m (2H), 4,51 s (1H), 4,60 s (2H), 7,4 - 7,7 breit (1H,NH)

### Beispiel 13 (erfindungsgemäß)

140 g 4-Chlor-3-(n-propylamino)but-2-encarbonsäureethylester (Beispiel 3) werden in 216 g Toluol gelöst und 2 h unter Rückfluss erhitzt. Anschließend wird auf 20°C abgekühlt und das Lösungsmittel im Vakuum entfernt. Man erhält 100,3 g 4-(n-Propylamino)furan-2(5H)-on mit einer Reinheit von 86 % (dies entspricht 89% Ausbeute).
¹H-NMR (DMSO d₆, 298K) δ: 0,89 t (3H), 1,51 m (2H), 3,00 q (2H), 4,52 s (1H), 4,60 s (1H), 7,4 - 7,6 breit (1H,NH)

### Beispiel 14 (nicht erfindungsgemäß)

50 g 4-Chlor-3-(n-butylamino)but-2-encarbonsäureethylester (Beispiel 5) werden in 86 g Toluol gelöst und 2 h unter Rückfluss erhitzt. Anschließend wird auf 20°C abgekühlt und das Lösungsmittel im Vakuum entfernt. Man erhält 40,7 g 4-(n-Butylamino)furan-2(5H)-on mit einer Reinheit von 86 % (dies entspricht 99 % Ausbeute).
¹H-NMR (DMSO d₆, 298K) δ: 0,89 t (3H), 1,33 m (2H), 1,50 m (2H), 3,01 q (2H), 4,51 s (1H), 4,59 s (2H), 7,4 -7,7 breit (1H, NH)

### Beispiel 15 (nicht erfindungsgemäß)

140 g 4-Chlor-3-(iso-Butylamino)but-2-encarbonsäureethylester (Beispiel 6) werden in 216 g Toluol gelöst und 2 h unter Rückfluss erhitzt. Anschließend wird auf 20°C abgekühlt und das Lösungsmittel im Vakuum entfernt. Man erhält 104 g 4-(iso-Butylamino)furan-2(5H)-on mit einer Reinheit von 87 % (dies entspricht 92% Ausbeute).
¹H-NMR (DMSO d₆, 298K) δ: 0,89 d (6H), 1,79 m (1H), 2,84 t (2H), 4,52 s (1H), 4,61 s (2H), 7,4 - 7,7 breit (1H, NH)

### Beispiel 16 (nicht erfindungsgemäß)

157 g 4-Chlor-3-(Benzylamino)but-2-encarbonsäureethylester (Beispiel 8) werden in 259 g Toluol gelöst und 4 h unter Rückfluss erhitzt. Anschließend wird auf 20°C abgekühlt, der Feststoff abfiltriert und mit 100 ml Toluol gewaschen. Man erhält 109 g 4-(Benzylamino)furan-2(5H)-on mit einer Reinheit von 92 % (dies entspricht 86% Ausbeute).
¹H-NMR (DMSO d₆, 298K) δ: 4,27 d (2H), 4,58 s (1H), 4,67 s (2H), 7,26 - 7,45 m (5H), 7,9 - 8,2 breit (1H,NH)

### Beispiel 17 (nicht erfindungsgemäß)

18,8 g 4-Chlor-3-([[6-chlorpyridin-3-yl]methyl]amino)but-2-encarbonsäureethylester (Beispiel 9) werden in 86 g Toluol gelöst und 9 h unter Rückfluss erhitzt. Anschließend wird auf 20°C abgekühlt und das Lösungsmittel im Vakuum entfernt. Man erhält 16,1 g 4-([[6-chlorpyridin-3-yl]methyl]amino)furan-2(5H)-on mit einer Reinheit von 96 % (dies entspricht 87% Ausbeute).
¹H-NMR (CDCl₃, 298K) δ: 4,33 d (2H), 4,66 s (1H), 4,72 s (2H), 6,5 - 6,7 breit (1H,NH), 7,32 d (1H), 7,63 d (1H), 8,34 d (1H)

### Beispiel 18 (erfindungsgemäß)

280 g 4-Chlor-3-(2,2-Difluorethylamino)but-2-encarbonsäureethylester (Beispiel 10) werden in 709 g Toluol gelöst und 4 h unter Rückfluss erhitzt. Anschließend wird auf 20°C abgekühlt, der Feststoff abfiltriert und mit 100 ml Toluol gewaschen. Man erhält 183 g 4-(Difluorethylamino)furan-2(5H)-on mit einer Reinheit von 97 % (dies entspricht 96% Ausbeute).
¹H-NMR (DMSO d₆, 298K) δ: 3,44 - 3,59 m (2H), 4,65 s (1H), 4,77 s (2H), 6,60 t + 6,14 t + 6,28 t (1 H, CHF₂), 7,4 - 7,9 breit (1H,NH)

### Beispiel 19 (erfindungsgemäß)

30 g 4-(Methylamino)furan-2(5H)-on (Beispiel 11) werden in 450 ml Dimethoxyethan bei Raumtemperatur vorgelegt. Anschließend werden 12,6 g Natriumhydroxid zugesetzt und bei 40 °C werden 225 g einer 20%igen Lösung von 2-Chlor-5-(chlormethyl)pyridin in Dimethoxyethan zudosiert. Es wird noch weitere 6 h bei 50°C gerührt. Das Lösungsmittel wird weitgehend im Vakuum entfernt und der Rückstand mit 300 ml Wasser versetzt. Der Feststoff wird abfiltriert, mit 150 ml Wasser gewaschen und im Vakuum getrocknet. Man erhält 58,7 g 4-([[6-chlorpyridin-3-yl]methyl](methyl)amino)furan-2(5H)-on mit einer Reinheit von 94 % (dies entspricht 87% Ausbeute).
¹H-NMR (DMSO d₆, 298K) δ: 2,88 s (3H), 4,47 s (2H), 4,74 s (1H), 4,89 s (2H), 7,52 d (1H), 7,78 d (1H), 8,37 s (1H)

### Beispiel 20 (nicht erfindungsgemäß)

6,3 g 4-(Benzylamino)furan-2(5H)-on (Beispiel 16) werden in 75 ml Dimethoxyethan bei Raumtemperatur vorgelegt. Anschließend werden 1,1 g Natriumhydroxid zugesetzt und bei 40 °C werden 25,5 g einer 20%igen Lösung von 2-Chlor-5-(chlormethyl)pyridin in Dimethoxyethan zudosiert. Es wird noch weitere 6 h bei 50°C gerührt. Das Lösungsmittel wird weitgehend im Vakuum entfernt und der Rückstand mit 50 ml Wasser und 50 ml Methylenchlorid versetzt. Die organische Phase wird abgetrennt, die wässrige Phase wird nochmals mit 50 ml Methylenchlorid extrahiert. Die vereinigten Methylenchloridphasen werden über Natriumsulfat getrocknet und das Lösungsmittel im Vakuum entfernt. Man erhält 8 g 4-{Benzyl[(6-chlorpyridin-3-yl)methyl]amino}furan-2(5H)-on (dies entspricht 85% Ausbeute).
1H NMR (Acetonitrile-*d*₃) δ: 4.40 (s, 4 H) 4.71 (s, 1 H) 4.87 (s, 2 H) 7.23 (d, *J*=7.37 Hz, 2 H) 7.28 - 7.33 (m, 1 H) 7.33 - 7.38 (m, 3 H) 7.61 (dd, *J*=8.25, 2.64 Hz, 1 H) 8.20 (d, *J*=2.20 Hz, 1 H)

### Beispiel 21 (nicht erfindungsgemäß)

6,3 g 4-(Benzylamino)furan-2(5H)-on (Beispiel 16) werden in 75 ml Dimethoxyethan bei Raumtemperatur vorgelegt. Anschließend werden 1,3 g Natriumhydroxid zugesetzt und bei 40 °C werden 34,4 g einer 15%igen Lösung von 2-Chlor-5-(chlormethyl)-1,3-thiazol in Dimethoxyethan zudosiert. Es wird noch weitere 6 h bei 50°C gerührt. Das Lösungsmittel wird weitgehend im Vakuum entfernt und der Rückstand mit 50 ml Wasser und 50 ml Methylenchlorid versetzt. Die organische Phase wird abgetrennt, die wässrige Phase wird nochmals mit 50 ml Methylenchlorid extrahiert. Die vereinigten Methylenchloridphasen werden über Natriumsulfat getrocknet und das Lösungsmittel im Vakuum entfernt. Man erhält 6,9 g 4-{Benzyl[(2-chlor-1,3-thiazol-5-yl)methyl]amino}furan-2(5H)-on (dies entspricht 71 % Ausbeute).

### Beispiel 22 (nicht erfindungsgemäß)

6,3 g 4-(Benzylamino)furan-2(5H)-on (Beispiel 16) werden in 75 ml Dimethoxyethan bei Raumtemperatur vorgelegt. Anschließend werden 1,1 g Natriumhydroxid zugesetzt und bei 40 °C werden 4 g Benzylchlorid zudosiert. Es wird noch weitere 7 h bei 50°C gerührt. Das Lösungsmittel wird weitgehend im Vakuum entfernt und der Rückstand mit 50 ml Wasser und 50 ml Methylenchlorid versetzt. Die organische Phase wird abgetrennt, die wässrige Phase wird nochmals mit 50 ml Methylenchlorid extrahiert. Die vereinigten Methylenchloridphasen werden über Natriumsulfat getrocknet und das Lösungsmittel im Vakuum entfernt. Man erhält 5,6 g 4-(Dibenzylamino)furan-2(5H)-on (dies entspricht 68 % Ausbeute).

## Patentansprüche

1. Verfahren zum Herstellen von Verbindungen der Formel (IVa) 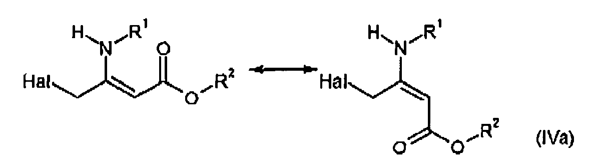 in welcher
R¹ für Methyl, Ethyl, n-Propyl, n-Prop-2-enyl, n-Prop-2-inyl, Cyclopropyl, Methoxyethyl, 2-Fluorethyl, oder 2,2-Difluorethyl steht,
R² für Methyl oder Ethyl steht und
Hal für Chlor stcht,
in dem ein 4-Halogenacetessigester der Formel (II) 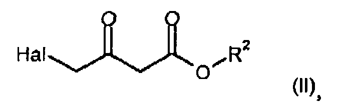 mit einem Amin der Fonnel (IIIa) 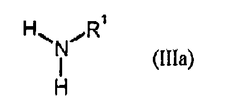 worin in den Formeln (II) und (IIIa) R¹, R² und Hal die oben genannte Bedeutung haben,
in Toluol oder einer Mischung aus Toluol und Ethanol und bei Temperaturen von 10°C bis 40°C bei Normaldruck umgesetzt wird, wobei anschließend Toluol oder eine Mischung aus Toluol und Ethanol durch Abdestillieren oder im Vakuum im Temperaturbereich von 20 °C bis 35 °C entfernt wird.

2. Das Verfahren nach Anspruch 1, in dem eine Lewis-Säure, die ausgewählt ist aus einer Gruppe bestehend aus Essigsäure, p-Toluolsulfonsäure und Trifluoressigsäure, der Reaktion zugesetzt wird.

3. Verbindungen der Formel (IVa) 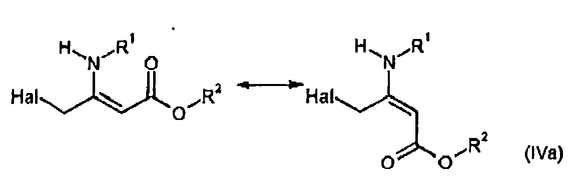 worin
R¹ für Methyl, Ethyl, n-Propyl, n-Prop-2-enyl, n-Prop-2-inyl, Cyclopropyl, Methoxyethyl, 2-Fluorethyl, oder 2,2-Difluorethyl steht,
R² für Methyl oder Ethyl steht; und
Hal für Chlor steht.

4. Verwendung einer Verbindung der Formel (IVa), die wie in Anspruch 1 definiert und hergestellt ist, zur Herstellung eines 4-Aminobut-2-enolids der Formel (I) 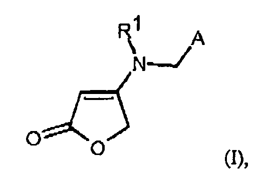 in der
A für den Rest 6-Fluor-pyrid-3-yl, 6-Chlor-pyrid-3-yl 6-Brom-pyrid-3-yl, 2-Chlor-1,3-thiazol-5-yl, 5-Fluor-6-chlor-pyrid-3-yl, 5-Fluor-6-brom-pyrid-3-yl oder 5,6-Dichlor-pyrid-3-yl steht; und
R¹ für Methyl, Ethyl, n-Propyl, n-Prop-2-enyl, n-Prop-2-inyl, Cyclopropyl, Methoxyethyl, 2-Fluorethyl, oder 2,2-Difluor-ethyl steht,
in dem die Verbindung der Fonnel (IVa) in Gegenwart eines Lösungsmittels thermisch zu einer Verbindung der Formel (Va) cyclisiert wird 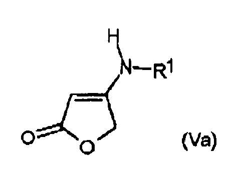 und die Verbindung der Formel (Va) in einem letzten Schritt mit einer Vorbindung der Formel (VIa)
A-CH₂-E (VIa)
umgesetzt wird, wobei
A die oben genannte Bedeutung hat, und
E für eine Abgangsgruppe steht, die ausgewahlt ist unter Chlor, Brom, Jod, Mesylat, Tosylat oder SO₂Me steht.

5. Die Verwendung nach Anspruch 4, **dadurch gekennzeichnet, dass** die thermische Cyclisierung der Verbindung der Formel (IVa) zur Verbindung der Formel (Va) bei 40°C bis 150°C erfolgt.

## Claims

1. Process for preparing compounds of the formula (IVa) 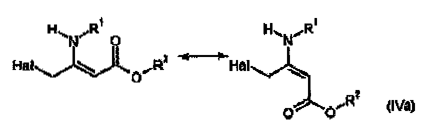 in which
R¹ is methyl, ethyl, n-propyl, n-prop-2-enyl, n-prop-2-ynyl, cyclopropyl, methoxyethyl, 2-fluoroethyl or 2,2-difluoroethyl,
R² is methyl or ethyl and
Hal is chlorine,
in which a 4-haloacetoacetic ester of the formula (II) 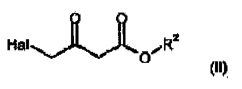 is reacted with an amine of the formula (IIIa) 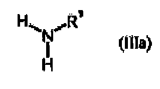 in which R¹, R² and Hal in the formulae (II) and (IIIa) are each as defined above
in toluene or a mixture of toluene and ethanol and at temperatures of 10°C to 40°C at standard pressure, and then toluene or a mixture of toluene and ethanol is removed by distillative removal or under reduced pressure within the temperature range from 20°C to 35°C.

2. Process according to Claim 1, in which a Lewis acid selected from a group consisting of acetic acid, p-toluenesulphonic acid and trifluoroacetic acid is added to the reaction.

3. Compound of the formula (IVa) 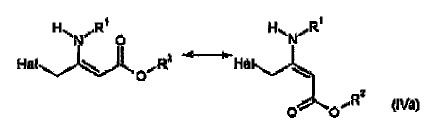 in which
R¹ is methyl, ethyl, n-propyl, n-prop-2-enyl, n-prop-2-ynyl, cyclopropyl, methoxyethyl, 2-fluoroethyl or 2,2-difluoroethyl,
R² is methyl or ethyl and
Hal is chlorine.

4. Use of a compound of the formula (IVa) which is defined and prepared as in Claim 1 for preparation of a 4-aminobut-2-enolide of the formula (I) 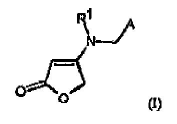 in which
A is the 6-fluoropyrid-3-yl, 6-chloropyrid-3-yl, 6-bromopyrid-3-yl, 2-chloro-1,3-thiazol-5-yl, 5-fluoro-6-chloropyrid-3-yl, 5-fluoro-6-bromo-pyrid-3-yl or 5,6-dichloropyrid-3-yl radical; and
R¹ is methyl, ethyl, n-propyl, n-prop-2-enyl, n-prop-2-ynyl, cyclopropyl, methoxyethyl, 2-fluoroethyl or 2,2-difluoroethyl,
in which the compound of the formula (IVa) is cyclized thermally in the presence of a solvent to give a compound of the formula (Va) 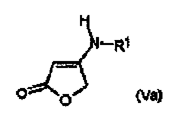 and the compound of the formula (Va) is reacted in a last step with a compound of the formula (VIa)
**A-CH₂-E** **(VIa)**
where
A is as defined above and
E is a leaving group selected from chlorine, bromine, iodine, mesylate, tosylate and SO₂Me.

5. Use according to Claim 4, **characterized in that** the thermal cyclization of the compound of the formula (IVa) to the compound of the formula (Va) is effected at 40°C to 150°C.

## Revendications

1. Procédé pour la préparation de composés de formule (IVa) 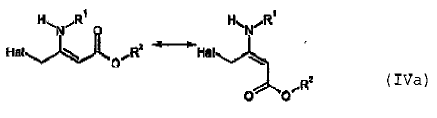 dans laquelle
R¹ représente le groupe méthyle, éthyle, n-propyle, n-prop-2-ényle, n-prop-2-ynyle, cyclopropyle, méthoxyéthyle, 2-fluoroéthyle, ou 2,2-difluoroéthyle,
R² représente le groupe méthyle ou éthyle et
Hal représente un atome de chlore,
dans lequel on fait réagir un ester d'acide 4-halogéno-acétoacétique de formule (II) 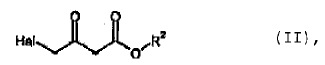 avec une amine de formule (IIIa) 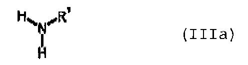 dans lequel dans les formules (II) et (IIIa) R¹, R² et Hal ont les significations données ci-dessus,
dans du toluène ou un mélange de toluène et d'éthanol et à des températures de 10 °C à 40 °C sous la pression normale, en éliminant ensuite le toluène ou un mélange de toluène et d'éthanol par distillation ou sous vide dans la plage de température de 20 °C à 35 °C.

2. Procédé selon la revendication 1, dans lequel on ajoute au mélange réactionnel un acide de Lewis, qui est choisi dans un groupe constitué par l'acide acétique, l'acide p-toluènesulfonique et l'acide trifluoroacétique.

3. Composés de formule (IVa) 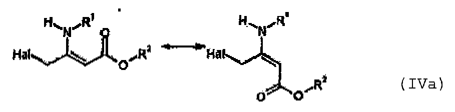 dans laquelle
R¹ représente le groupe méthyle, éthyle, n-propyle, n-prop-2-ényle, n-prop-2-ynyle, cyclopropyle, méthoxyéthyle, 2-fluoroéthyle, ou 2,2-difluoroéthyle,
R² représente le groupe méthyle ou éthyle ; et
Hal représente un atome de chlore.

4. Utilisation d'un composé de formule (IVa), qui est défini et préparé comme dans la revendication 1, pour la préparation d'un 4-aminobut-2-énolide de formule (I) 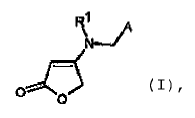 dans laquelle
A représente le radical 6-fluoro-pyrid-3-yle, 6-chloro-pyrid-3-yle, 6-bromo-pyrid-3-yle, 2-chloro-1,3-thiazol-5-yle, 5-fluoro-6-chloro-pyrid-3-yle, 5-tluoro-6-bromo-pyrid-3-yle ou 5,6-dichloro-pyrid-3-yle ; et
R¹ représente le groupe méthyle, éthyle, n-propyle, n-prop-2-ényle, n-prop-2-ynyle, cyclopropyle, méthoxyéthyle, 2-fluoroéthyle, ou 2,2-difluoroéthyle,
dans lequel on soumet à une cyclisation thermique le composé de formule (IVa) en présence d'un solvant, pour aboutir à un composé de formule (Va) 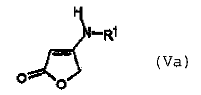 et dans une dernière étape on fait réagir le composé de formule (Va) avec un composé de formule (VIa)
A-CH₂-E (VIa)
où
A a la signification donnée plus haut, et
E représente un groupe partant, qui est choisi parmi chloro, bromo, iodo, mésylate, tosylate ou SO₂Me.

5. Utilisation selon la revendication 4, **caractérisée en ce que** la cyclisation thermique du composé de formule (IVa) en le composé de formule (Va) s'effectue à une température de 40 °C à 150 °C.
